# FASCICULE DE BREVET EUROPEEN

(11) **EP 2 339 924 B1**
(45) Date de publication et mention de la délivrance du brevet: **02.09.2015**
(21) Numéro de dépôt: 09772755.6
(22) Date de dépôt: 03.07.2009
(51) Int. Cl.: A23C 9/123, A23C 19/032, C12N 1/20

(54) **PROCÉDÉ DE PRÉPARATION D'UN LEVAIN À PARTIR DE LAIT CRU**
VERFAHREN ZUR HERSTELLUNG EINES FERMENTATIONSMITTELS AUS UNPASTEURISIERTER MILCH
METHOD FOR PREPARING A LEAVEN FROM UNPASTEURISED MILK

(30) Priorité: 04.07.2008 FR 0803808
(43) Date de publication de la demande: 06.07.2011
(73) Titulaire: Dumarché, Claude, 17340 Chatelaillon Plage (FR); Leclercq, Sébastien, 12100 Millau (FR)
(72) Inventeur: Dumarché, Claude, 17340 Chatelaillon Plage (FR); Leclercq, Sébastien, 12100 Millau (FR)
(74) Mandataire: Cabinet BARRE LAFORGUE & associés
(86) Numéro de dépôt international: PCT/FR2009/051314
(87) Numéro de publication internationale: WO 2010/001073

(56) Documents cités:
- EP-A- 0 505 164
- WO-A-2005/110107
- JP-A- 2005 237 238
- WOUTERS JAN T M ET AL: "Microbes from raw milk for fermented dairy products" INTERNATIONAL DAIRY JOURNAL, vol. 12, no. 2-3, 2002, pages 91-109, XP002513969 ISSN: 0958-6946
- PARENTE D: "Diversity and dynamics of microbial communities in natural and mixed starter cultures" AUSTRALIAN JOURNAL OF DAIRY TECHNOLOGY, vol. 61, no. 2, juillet 2006 (2006-07), pages 108-115, XP009111909
- BABEL F J: "Antibiosis by lactic culture bacteria." JOURNAL OF DAIRY SCIENCE, vol. 60, no. 5, 1977, pages 815-821, XP002513970 PURDUE UNIV., WEST LAFAYETTE, INDIANA 47907, USA

## Description

L'invention concerne un procédé de préparation d'un levain à partir de lait cru.

Les directives sanitaires visant à réduire les risques d'intoxication alimentaire en éliminant les flores pathogènes dans les produits laitiers obligent les professionnels de l'industrie laitière à prendre, dans les procédés de production et de collecte des laits, des mesures drastiques d'hygiène, notamment des mesures de désinfection et de décontamination des locaux de traite, des mamelles des animaux, des dispositifs de traite et des dispositifs de stockage des laits. En outre, ces directives ont conduit à la généralisation des procédés de stockage réfrigéré du lait pendant 48 h à 72 h à la ferme, notamment à une température de l'ordre de +4°C. A cet égard, le document Michel V. et al, (2001), Lait, 81, pp575-592, décrit l'impact de conditions sanitaires variées, adoptées lors de la production de laits de vache, sur la diversité biologique de la flore microbienne de ces laits crus.

Cependant le déclin de la quantité moyenne des flores pathogènes dans les productions laitières, résultant de la mise en oeuvre de ces méthodes, s'accompagne de la diminution sensible, dans les laits crus, de la quantité moyenne des flores utiles pour la fermentation des laits et pour l'affinage de produits laitiers issus de laits crus, notamment des fromages.

En outre, pour satisfaire aux exigences sanitaires, visant à limiter les flores pathogènes dans les laits crus, et en même temps bénéficier d'une flore numériquement importante et adaptée pour réaliser la fermentation lactique du lait, l'industrie laitière préconise l'utilisation de ferments, appelés ferments issus de co-culture, dont la composition est parfaitement définie et aisément reproductible. Ces ferments issus de co-culture sont constitués d'une souche ou d'un petit nombre de souches microbiologiques pures, sont élaborés par isolement et clonage de ces souches pures dans des laboratoires de recherche, et sont donc exempts de germes potentiellement pathogènes. Ces ferments issus de co-culture sont, par exemple, constitués d'une combinaison d'une ou plusieurs souche(s) bactérienne(s) acidifiante(s) et d'une ou plusieurs souche(s) bactérienne(s) non acidifiantes adaptées pour le typage des produits obtenus à partir de ces ferments.

Cependant, le développement des propriétés organoleptiques et les qualités sensorielles des fromages au lait cru, ainsi que leur typicité caractéristique d'une zone géographique ou géomorphologique, appelée terroir, est une conséquence directe de l'utilisation de laits crus présentant une flore naturellement diversifiée, elle-même caractéristique de ce terroir. En effet, les qualités organoleptiques originales des fromages au lait cru proviennent de l'expression d'un grand nombre de molécules odorantes et gustatives de faible poids moléculaire, produites lors de dégradations enzymatiques de divers constituants, notamment protéiques, du lait. Ainsi, la richesse et la diversité microbiologique des laits crus permettent l'expression d'équipements enzymatiques eux-mêmes riches et diversifiés qui sont à l'origine de la production, dans les milieux de fermentation, notamment de fermentation lactique, et dans les milieux d'affinage, de molécules à l'origine des saveurs et des odeurs caractéristiques des fromages au lait cru.

On connaît déjà différents procédés permettant de préparer un concentré de cellules bactériennes à partir de souches microbiologiquement pures.

Dans un premier type de solution connue (cf. par exemple GB 1 205 733), on prépare un concentré de cellules bactériennes à partir d'une souche structurellement caractérisée et commercialement disponible de *Streptococcus cremoris.* Une telle solution ne permet pas de préparer, à partir de lait cru, un levain présentant une flore microbiologique riche et diversifiée, dont la composition microbiologique est représentative de la zone géographique et des pratiques de production du lait cru.

Un deuxième type de solution connue (WO 2006/067136) semblable au précédent est un procédé de préparation rapide d'un concentré de bactéries lactiques notamment de *Lactococcus lactis,* ssp *cremoris* utilisant un extrait de levure à titre d'accélérateur de croissance. Une telle solution ne permet pas non plus de préparer, à partir de lait cru, un levain fortement diversifié.

L'invention vise à pallier ces inconvénients en proposant un procédé de préparation, à partir de lait cru, d'un levain formé d'un grand nombre d'espèces microbiologiques, notamment bactériennes, différentes.

L'invention vise à proposer un procédé de préparation d'un levain dont la composition comprend un grand nombre d'espèces microbiologiques différentes et regroupe l'essentiel de la flore fromagère utile du lait cru.

L'invention vise aussi à proposer un tel procédé ne faisant appel à aucun traitement du lait cru par des compositions antiseptiques, antibiotiques, bactériostatiques ou par éléments chimiques susceptibles d'inhiber ou stimuler la croissance de la flore du lait cru.

L'invention vise également à proposer un tel procédé ne faisant appel à aucun ajout volontaire de micro-organisme.

L'invention vise également et plus particulièrement à proposer un tel procédé qui tend à se conformer aux contraintes de sécurité sanitaire auxquelles sont soumis les producteurs de lait et l'industrie laitière.

L'invention vise de surcroît à proposer un tel procédé qui préserve les habitudes de travail des personnels, qui soit facile à utiliser, et qui n'implique pour sa mise en oeuvre que peu de manipulations.

L'invention vise également à proposer un tel procédé réalisé à partir de moyens et de dispositifs peu onéreux.

Dans toute la suite :
- le terme « lait cru» désigne non seulement un lait qui n'a subi aucun traitement à une température supérieure à la température du lait en sortie de mamelle, mais aussi plus largement un lait qui n'a subi aucun traitement physique ou thermique susceptible d'éliminer ou de détruire des germes microbiologiques. On sait que le lait cru produit par des animaux sains est sensiblement stérile en sortie de la mamelle, et que la flore présente dans le lait cru après la traite est constituée de micro-organismes exogènes caractéristiques de l'environnement de production de ce lait cru.
- le terme « flore utile » désigne l'ensemble de la flore du lait cru participant à la maturation, à l'acidification et à la coagulation du lait cru et au développement de la flore de surface. La flore utile, aussi appelée flore fromagère ou encore flore technologique, comprend par exemple, et à titre non limitatif, les lactocoques, notamment *Lactococcus cremoris*, *Lactococcus diacetylactis,* les lactobacilles et les bactéries du genre *Leuconostoc.*
- le terme «flore néfaste» désigne l'ensemble de la flore du lait contribuant à l'altération soit des propriétés organoleptiques (flore d'altération), soit de la qualité sanitaire (flore pathogène) du produit laitier final. La flore d'altération comprend notamment la famille de *Pseudomonas* et des bactéries coliformes en général. La flore pathogène comprend les Staphylocoques à coagulase positive, notamment *Staphylococcus aureus,* la famille des Listéria, notamment *Listeria monocytogenes,* les salmonelles et *Escherichia coli.*

L'invention concerne donc un procédé de préparation d'un levain selon la revendication 1.

Les laits crus produits dans des exploitations laitières respectant les nonnes sanitaires actuellement en vigueur, présentent une densité microbiologique globalement faible, notamment une densité microbiologique sensiblement inférieure à celle des laits crus produits dans des exploitations laitières ne respectant pas ces directives sanitaires. Cependant ces laits crus, présentant une densité microbiologique globalement faible, contiennent néanmoins une flore microbiologique suffisamment nombreuse et diversifiée pour permettre l'élaboration de levains, présentant une flore diversifiée, susceptibles de typer les produits obtenus à partir de ces levains.

Les inventeurs on constaté que, quelle que soit leur origine et leur nature, la plupart des laits crus produits dans ces exploitations laitières peuvent être avantageusement enrichis en flore utile par un traitement de stabilisation comprenant au moins une étape de fermentation stabilisante dans laquelle on interrompt la fermentation lactique acidifiante lorsque le pH du milieu de fermentation atteint une valeur comprise entre 4,6 et 6,0 notamment une valeur comprise entre 4,9 et 5,6-, en particulier une valeur de l'ordre de 5,2.

D'autre part, ce traitement de stabilisation permet non seulement d'augmenter le titre microbiologique du milieu de fermentation, mais également de favoriser le développement, dans le milieu de fermentation, des flores utiles aux dépends des flores d'altération et des flores pathogènes dudit milieu de fermentation.

Les inventeurs ont constaté avec surprise que l'interruption de la fermentation lactique acidifiante lorsque le pH du milieu de fermentation atteint une valeur comprise entre 4,6 et 6,0 -notamment une valeur comprise entre 4,9 et 5,6-, en particulier une valeur de l'ordre de 5,2, permet, quelle que soit l'origine et la nature du lait cru, de favoriser la croissance des micro-organismes hétérofermentaires de la flore utile du milieu de fermentation tout en limitant la croissance des micro-organismes acidophiles stricts. En outre, cette interruption permet de modifier, de façon différentielle, les taux de croissance respectifs des différentes flores présentes dans le milieu de fermentation, et d'enrichir le milieu de fermentation en flores fromagères diversifiées issues du lait cru.

Les inventeurs ont observé qu'il est possible de choisir les laits crus susceptibles d'être utilisés pour la préparation d'un levain selon l'invention en réalisant une fermentation lactique acidifiante à une température, dite température de sélection, adaptée pour permettre la croissance de la flore du lait cru, ladite température de sélection étant comprise entre 20°C et 42°C, notamment de l'ordre de 27°C, et en sélectionnant les laits fermentés dont le pH atteint la valeur de 5,5 en plus de 15 h, et la valeur minimale de 5,0 en moins de 48 h. Ainsi, on sélectionne des laits crus dont la flore est diversifiée et adaptée pour réaliser une acidification lente du lait cru à au moins une température de sélection.

Avantageusement et selon l'invention, on réalise cette étape d'identification et de sélection des laits crus susceptibles d'être utilisés pour la préparation d'un levain, à une température de sélection de l'ordre de 27°C, notamment à la température de 27°C, de façon à sélectionner les laits crus riches en flores mésophiles présentant cette double propriété d'acidification lente et stabilisante. Le lait ainsi sélectionné est un lait cru riche en flores mésophiles qui, à la température de sélection de 27°C, permettent une acidification lente d'un milieu de fermentation d'une première étape de fermentation stabilisante selon l'invention.

On peut qualifier un tel procédé selon l'invention de procédé « bionatif ». En effet, il tire avantage de la biodiversité du lait et en particulier de la biodiversité du lait cru natif. Par extension on qualifiera de « levain natif» un tel levain selon l'invention obtenu par un procédé « bionatif » selon l'invention.

Avantageusement et selon l'invention, on réalise ladite première étape de fermentation stabilisante sans ajout ni retranchement artificiel d'espèces microbiologiques au/du lait cru. En particulier, on réalise chaque étape de fermentation stabilisante sans ajout ni retranchement artificiel d'espèces microbiologiques au/du lait cru.

Avantageusement et selon l'invention, on réalise chaque étape de fermentation stabilisante à une température prédéterminée comprise entre 18°C et 35°C, notamment à une température prédéterminée comprise entre 23°C et 30°C, particulièrement à une température prédéterminée de l'ordre de 27°C. Avantageusement, on réalise chaque étape de fermentation stabilisante à une température prédéterminée de 27°C. Les inventeurs ont observé qu'une température prédéterminée comprise entre 18°C et 35°C, notamment une température prédéterminée comprise entre 23°C et 30°C, particulièrement une température prédéterminée de l'ordre de 27°C est adaptée pour favoriser la croissance des micro-organismes mésophiles dans le milieu de fermentation, notamment des micro-organismes mésophiles hétérofermentaires de la flore utile. Plus particulièrement, une telle température prédéterminée d'une étape de fermentation stabilisante est adaptée pour permettre la diminution de la proportion de la flore d'altération par rapport à la flore totale dans le milieu de fermentation. Encore plus particulièrement, une telle température prédéterminée d'une étape de fermentation stabilisante est adaptée pour favoriser la flore thermo-tolérante présentant des conditions optimales de croissance proches des conditions de croissance de la flore thermophile et augmenter la biodiversité de la flore du milieu de fermentation.

Avantageusement et selon l'invention, on interrompt la première étape de fermentation stabilisante après une durée de fermentation lactique acidifiante du milieu de fermentation comprise entre 12 h et 36 h, en particulier entre 17 h et 24 h.

Les inventeurs ont observé que l'acidification lente d'un milieu de fermentation lors d'une première étape de fermentation stabilisante, en particulier une acidification permettant de diminuer, le pH du milieu de fermentation à une valeur comprise entre 4,6 et 6,0 -notamment à une valeur comprise entre 4,9 et 5,6- en particulier à une valeur de l'ordre de 5,2, en plus de 12 h et en moins de 36 h, notamment en plus de 17 h et en moins de 24 h, permet d'amplifier la flore constitutive du milieu de fermentation.

En l'espèce, les inventeurs ont observé que l'amplification de la flore constitutive du milieu de fermentation lors d'une première étape de fermentation stabilisante ne conduit pas à une sélection d'un petit nombre d'espèces microbiologiques du milieu de fermentation (ni donc à une réduction du nombre d'espèces microbiologiques présentes dans ledit milieu de fermentation), mais au contraire à la croissance de l'essentiel des espèces microbiennes initialement présentes dans le milieux de fermentation.

En outre, une telle étape de fermentation stabilisante d'un milieu de fermentation formé d'un lait cru permet de modifier les proportions relatives des différentes espèces microbiologiques présentes dans le milieu de fermentation, tout en conservant l'essentiel des espèces microbiologiques constitutives de la flore utile nécessaire à l'élaboration d'un levain, dont la biodiversité est représentative de la biodiversité de la flore microbiologique constitutive du lait cru ayant servi à sa préparation.

Avantageusement et selon l'invention, le milieu de fermentation de la première étape de fermentation stabilisante est un lait cru issu de mammifère, notamment un lait cru choisi dans le groupe formé du lait cru issu de vache, du lait cru issu de brebis, du lait cru issu de chèvre et du lait cru issu de bufflonne. En particulier, le milieu de fermentation d'une première étape de fermentation stabilisante est un lait cru issu d'animaux de la classe des mammifères, à l'exception des laits issus d'animaux faisant partie de l'ordre des monotrèmes.

En effet, les inventeurs ont observé que l'acidification lente d'un milieu de fermentation formé d'un lait cru, lors d'une première étape de fermentation stabilisante, en particulier une acidification permettant de diminuer le pH du milieu de fermentation à une valeur comprise entre 4,6 et 6,0 -notamment à une valeur comprise entre 4,9 et 5,6-, en particulier à une valeur de l'ordre de 5,2, en plus de 12 h et en moins de 36 h, notamment en plus de 17 h et en moins de 24 h, permet d'amplifier l'essentiel de la flore -notamment de la flore utile- constitutive du lait cru.

En particulier, une telle étape de fermentation stabilisante lente d'un milieu de fermentation formé d'un lait cru permet de diminuer la proportion de la flore d'altération dans des laits riches en flores d'altération au profit de flores utiles diversifiées dans le milieu de fermentation à pH sensiblement compris entre 4,6 et 6,0 -notamment à une valeur comprise entre 4,9 et 5,6-, en particulier à une valeur de l'ordre de 5,2 obtenu à l'issue d'une première étape de fermentation stabilisante d'un traitement de stabilisation.

Ce résultat surprenant n'a pas d'explication claire. Les inventeurs pensent qu'il pourrait être dû, au moins en partie, au fait que l'acidification lente d'un milieu de fermentation formé d'un lait cru génère, au cours de l'acidification lente, des conditions physico-chimiques variées susceptibles de permettre la croissance de la flore utile et en permettant l'établissement d'un équilibre stable entre toutes les espèces microbiologiques constitutives du milieu de fermentation.

En outre, l'amplification de l'essentiel des espèces microbiologiques de la flore utile dans le milieu de fermentation lors d'une première étape de fermentation stabilisante, s'accompagne aussi d'une augmentation de la proportion des bactéries coliformes, notamment des bactéries pathogènes du genre *Escherichia coli,* dans ledit milieu de fermentation. Les inventeurs ont observé avec surprise que la proportion de bactéries coliformes, notamment des bactéries pathogènes du genre *Escherichia coli* du milieu de fermentation à l'issue de la première étape de fermentation stabilisante, décroit dans les milieux de fermentation formés à l'issue des étapes ultérieures de fermentation stabilisante.

Avantageusement et selon l'invention, le milieu de fermentation de la première étape de fermentation stabilisante comprend des micro-organismes vivants à une concentration comprise entre 5 10³ et 5 10⁵ micro-organismes vivants/mL. Particulièrement, il est possible que le milieu de fermentation de la première étape de fermentation stabilisante soit un lait cru propre, c'est-à-dire comprenant de l'ordre de 10⁴ micro-organismes vivants/mL. Il est aussi possible que le milieu de fermentation de la première étape de fermentation stabilisante soit un lait cm comprenant plus de 10⁵ micro-organismes vivants/mL, - notamment de l'ordre de 2 10⁵ micro-organismes vivants/mL-. Il est aussi possible que le milieu de fermentation, formé d'un lait cru, de la première étape de fermentation stabilisante soit un lait cru ultra-propre comprenant moins de 10⁴ micro-organismes vivants/mL -notamment de l'ordre de 5 10³ micro-organismes vivants/mL-. De façon générale, le milieu de fermentation, formé d'un lait cru, de la première étape de fermentation stabilisante est un lait cru exempt de micro-organismes pathogènes des genres *Listeria* et *Salmonella.*

Avantageusement et selon l'invention, on réalise au moins deux -notamment six- étapes successives de fermentation stabilisante, chaque étape de fermentation stabilisante étant interrompue (une interruption de la fermentation étant réalisée entre les deux étapes successives). En particulier, on réalise après une première étape de fermentation stabilisante d'un lait cru, une succession de plusieurs étapes ultérieures de fermentation stabilisante d'un traitement selon l'invention de façon à appauvrir le milieu de fermentation en flore d'altération, en flores coliformes et en bactéries du genre *Escherichia coli* et de façon à enrichir le milieu de fermentation en flore utile. Particulièrement, les inventeurs ont observé que la réalisation de six étapes de fermentation stabilisante successives à partir d'un lait cru permet d'obtenir, à l'issue des six étapes de fermentation stabilisante, un milieu de fermentation dont la valeur de pH est sensiblement comprise entre 4,6 et 6,0 -notamment comprise entre 4,9 et 5,6-, en particulier de l'ordre de 5,2, contenant une proportion de l'ordre de quelques micro-organismes, notamment de 1 à 10 micro-organismes, appartenant à la flore d'altération pour 10⁶ micro-organismes totaux. En variante; et avantageusement, on réalise au moins dix étapes de fermentation stabilisante successives à partir d'un lait cru le nombre d'étapes de fermentation stabilisante successives étant adapté pour obtenir une telle proportion de la flore d'altération par rapport à la flore totale.

Or, les inventeurs ont constaté de façon surprenante que l'accroissement de la proportion des bactéries coliformes, notamment des bactéries pathogènes du genre *Escherichia coli,* par rapport à la flore totale, de façon concomitante à la diminution de la proportion de la flore d'altération, dans le milieu de fermentation formé d'un lait cru d'une première étape de fermentation stabilisante, est une caractéristique propre à cette première étape de fermentation stabilisante d'un milieu de fermentation formé d'un lait cru. En effet, lors d'étapes ultérieures de fermentation stabilisante d'un traitement de stabilisation, la proportion des bactéries coliformes, notamment des bactéries pathogènes du genre *Escherichia coli* par rapport à la flore totale, diminue dans les milieux de fermentation successifs des étapes ultérieures de fermentation stabilisante, jusqu'à atteindre, par exemple, une valeur inférieure à 0,01%, notamment de l'ordre de 0,001%, voire même une quantité non-détectable de germe pathogène du genre *Escherichia coli.* Ainsi dans le milieu de fermentation d'une étape ultérieure de fermentation stabilisante d'un traitement de stabilisation, le nombre total de micro-organismes atteint une valeur de l'ordre de 2 10⁹ micro-organismes/mL, tandis que la proportion des bactéries du genre *Escherichia coli* par rapport à la flore totale, diminue jusqu'à atteindre, une valeur inférieure à 0,01%, notamment de l'ordre de 0,001 %, en particulier une valeur nulle.

Il est à noter que cette proportion des bactéries du genre *Escherichia coli* par rapport à la flore totale dans un milieu de fermentation d'une étape ultérieure de fermentation stabilisante est sensiblement inférieure à la proportion de ces mêmes bactéries du genre *Escherichia coli* par rapport à la flore totale dans un lait cru.

Les inventeurs ont observé que dans un milieu de fermentation d'une étape ultérieure de fermentation stabilisante, la proportion des bactéries *Escherichia coli* dans les milieux de fermentation d'étapes ultérieures de fermentation stabilisante successives diminue jusqu'à atteindre une proportion de l'ordre de quelques bactéries du genre *Escherichia coli* pour 10⁶ micro-organismes totaux.

Dans un milieu de fermentation d'une étape ultérieure de fermentation stabilisante, la proportion des conformes, des staphylocoques, des levures et des moisissures diminue lors des étapes successives de fermentation stabilisante jusqu'à ce que ces espèces ne soient plus détectables dans le milieu de fermentation d'une fermentation stabilisante ultime.

En particulier, les inventeurs ont observé que la réalisation d'une pluralité d'étapes successives de fermentation stabilisante conduit à la stabilisation d'un écosystème complexe formé des micro-organismes vivants constitutifs de la flore utile du lait cru et dont la composition est diversifiée et stable. De façon encore plus surprenante, les inventeurs ont constaté que la réalisation d'une pluralité d'étapes successives de fermentation stabilisante conduit à un écosystème sensiblement exempt de flore d'altération et de flore pathogène.

Ainsi, contrairement à un ferment issu de co-culture formé d'un petit nombre de souches de micro-organismes, notamment une, deux ou trois souche(s) de micro-organisme(s), qui ne se stabilise pas à l'identique lors de repiquages successifs et tend vers la sélection d'une souche unique qui se développe seule au sein de l'écosystème, le procédé selon l'invention permet la stabilisation d'un écosystème complexe, comprenant plusieurs dizaines de souches différentes de micro-organismes issus du lait cru, et comprenant en particulier l'essentiel de la flore utile dudit lait cru.

En particulier, les inventeurs ont observé que les cinétiques d'acidification des milieux de fermentation des étapes successives ultérieures de fermentation stabilisante d'un traitement de stabilisation présentent des profils sensiblement similaires. En particulier, ces profils présentent une phase de latence dont la durée est sensiblement conservée d'une étape à l'autre et une phase d'acidification rapide dont la valeur absolue maximale de la pente est sensiblement conservée d'une étape à l'autre.

Avantageusement et selon l'invention, on interrompt chaque étape de fermentation stabilisante par refroidissement du milieu de fermentation à une température inférieure à +4 °C, notamment à une température comprise entre 0°C et +4°C. Ainsi, on interrompt la fermentation lactique acidifiante dudit milieu de fermentation sans addition de substance visant à modifier les propriétés physico-chimiques, notamment les propriétés acido-basiques, et biologiques dudit milieu de fermentation. Une telle température comprise entre 0°C et +4°C est adaptée pour permettre un refroidissement rapide du milieu de fermentation issu de l'étape de fermentation et pour préserver sensiblement la totalité du potentiel de revivification des flores contenues dans ce milieu. Une telle température de refroidissement du milieu de fermentation est en outre adaptée pour éviter une croissance préférentielle des micro-organismes psychrophiles lors du refroidissement et de la conservation du milieu de fermentation.

Avantageusement et selon l'invention, on réalise au moins une étape, dite étape ultérieure de fermentation stabilisante, après la première étape de fermentation stabilisante, à partir d'un milieu de fermentation formé d'une quantité d'un milieu de culture liquide et d'une quantité d'un milieu de fermentation, dit milieu de fermentation précédent, obtenu à l'issue d'une étape précédente de fermentation stabilisante, dans un rapport volumique de la quantité de milieu de fermentation précédent sur la quantité de milieu de fermentation compris entre 0,5% et 25%, notamment compris entre 1%) et 5%, en particulier de l'ordre de 2 %.

Avantageusement et selon l'invention, le milieu de culture liquide est un jus lactosé, stérilisé et osmosé, contenant entre 4% et 20% en masse d'extrait sec, notamment 12% en masse d'extrait sec. Un jus lactosé, stérilisé et osmosé, selon la présente invention est un liquide stérilisé, contenant du lactose, dont les caractères salins et vitaminés sont équilibrés. En particulier, le milieu de culture liquide est choisi dans le groupe formé des laits d'origine naturelle, des laits standardisés et des laits reconstitués, susceptibles de subir une acidification par des bactéries lactiques. Plus particulièrement, le milieu de culture liquide est un lait d'origine naturelle choisi dans le groupe formé des laits pasteurisés, des laits thermisés et des laits micro-filtrés ou de tout autre lait d'origine naturelle.

Avantageusement et selon l'invention, le pH du milieu de culture liquide est compris entre 6,5 et 7,0, notamment sensiblement de l'ordre de 6,6. Les inventeurs ont observé qu'un milieu de culture formé d'un lait contenant entre 4% et 20% en masse d'extrait sec présente un pouvoir tampon suffisant pour permettre sensiblement la neutralisation de l'acidité du milieu de fermentation obtenu par mélange d'une quantité du milieu de culture liquide et d'une quantité du milieu de fermentation précédent dont la valeur de pH est comprise entre 4,6 et 6,0 - notamment comprise entre 4,9 et 5,6-, en particulier de l'ordre de 5,2, d'une étape de fermentation stabilisante précédente.

Avantageusement et selon l'invention, ledit milieu de fermentation précédent présente une valeur de pH comprise entre 4,6 et 6,0 - notamment entre 4,9 et 5,6-, en particulier de l'ordre de 5,2, et contient entre 5 10⁷ et 5 10⁹ micro-organismes/mL.

En outre, avantageusement et selon l'invention, on choisit le lait cru dans le groupe formé des laits crus contenant plus de 5.10³ micro-organismes mésophiles/mL, on soumet ce lait cru sélectionné à une première étape de fermentation stabilisante d'un traitement de stabilisation et on interrompt ladite étape lorsque le pH du milieu de fermentation atteint une valeur comprise entre 4,6 et 6,0 -notamment comprise entre 4,9 et 5,6-, en particulier une valeur de l'ordre de 5,2.

Avantageusement et selon l'invention, on choisit le lait cru dans le groupe formé des laits crus dont le pH est susceptible d'atteindre, lors d'une étape, dite étape de sélection, de fermentation stabilisante par fermentation lactique acidifiante du lait cru, aux températures de sélection de 27°C et de 40°C, une valeur de 5,5 en plus de 15 h, et une valeur minimum de 5,0 en moins de 48 h.

Avantageusement, on choisit le lait cru dans le groupe formé des laits crus dont le pH est susceptible d'atteindre, lors d'une étape, dite étape de sélection, de fermentation stabilisante par fermentation lactique acidifiante du lait cru, à deux températures de sélection comprises entre 20°C et 40°C, une valeur de 5,5 en plus de 15 h, et une valeur minimum de 5,0 en moins de 48 h

On réalise ladite étape de sélection du lait cru à deux températures, notamment 27°C et 40°C, de façon à sélectionner des laits crus riches en flores mésophiles et/ou thermophiles.

En outre, avantageusement et selon l'invention, on choisit, préalablement à la première étape de fermentation stabilisante d'un traitement de stabilisation, un lait cru dans le groupe formé des laits crus sensiblement exempts de micro-organisme pathogène, notamment du genre *Salmonella,* du genre *Listeria,* en particulier de l'espèce *Listeria monocytogenes.* En particulier, on sélectionne le lait cru dans le groupe formé des laits crus dont le taux de micro-organismes pathogènes est inférieur au seuil défini par les nonnes sanitaires, notamment des laits susceptibles de former sur milieu de culture solide moins d'une colonie de micro-organismes pathogènes pour 25 g de lait cru testé.

Avantageusement, on fabrique un levain exclusivement à partir de lait cru, sans ajout ni retranchement artificiel d'espèces microbiologiques au/du lait cru, par un procédé selon l'invention permettant de faire disparaître les staphylocoques, les levures et les moisissures du levain et permettant en outre de diminuer fortement la proportion de la flore d'hygiène, notamment la proportion de la flore coliforme, voire de la faire disparaître complètement du levain.

### Le levain obtenu par un procédé selon l'invention est un levain :

- comprenant plus de 10⁷ micro-organismes/mL, notamment entre 5.10⁷ et 5 10⁹ micro-organismes/mL, et
- comprenant de 5 à 100 espèces et sous-espèces microbiologiques différentes, notamment de 5 à 30 espèces et sous-espèces identifiables, en particulier de 5 à 12 espèces et sous-espèces microbiologiques identifiables, lesdites espèces et sous-espèces microbiologiques étant issues du lait cru uniquement par fermentation spontanée de celui-ci (sans ajout de levain ni retranchement artificiel d'espèces microbiologiques),
et dans lequel plus de 80% de ces espèces et sous-espèces microbiologiques contenues dans le levain sont issues de la flore utile du lait cru.

En variante, le levain est sensiblement exempt de coliformes, *d'Eschérichia coli* et de staphylocoques.

Avantageusement, le levain obtenu par un procédé selon l'invention contient des micro-organismes à une concentration supérieure à 10⁷ micro-organismes/mL, notamment une concentration comprise entre 5. 10⁷ et 5.10⁹ micro-organismes/mL, lesdits micro-organismes appartenant à des espèces et sous-espèces microbiologiques issues en totalité du lait cru, c'est-à-dire sans aucun ajout ni retranchement artificiel d'espèces microbiologiques au/du lait cru.

Avantageusement, le levain comprend de 5 à 100 espèces et sous-espèces microbiologiques différentes, notamment de 5 à 30 espèces et sous-espèces microbiologiques identifiables par des moyens biochimiques, microbiologiques ou génétiques connus en soi. En particulier, le levain comprend entre 5 et 12 espèces et sous-espèces microbiologiques identifiables qui sont issues du lait cru uniquement par fermentation spontanée de celui-ci, sans ajout ni retranchement artificiel d'espèces et sous-espèces microbiologiques au/du levain.

On réalise les analyses microbiologiques du levain obtenu par un procédé selon l'invention par tout moyen connu en soi. En particulier, on réalise ces analyses par des moyens microbiologiques de numération cellulaire, notamment par culture du levain sur des milieux de culture solide et dénombrement des colonies formées. On utilise à cet effet des milieux de culture solides dont la composition permet de sélectionner les différentes espèces microbiologiques en fonction des leurs exigences trophiques. On réalise en outre ces cultures à différentes températures adaptées pour répondre aux exigences thermiques optimales de croissance de la flore du levain. On réalise en outre ces cultures dans des conditions d'aération variées susceptibles de permettre la croissance de micro-organismes aérobies, de micro-organismes anaérobies stricts, de micro-organismes anaérobies aéro-tolérants.

On réalise les analyses de la composition microbiologique et de la biodiversité du levain obtenu par un procédé selon l'invention par des techniques moléculaires d'amplification, de séparation et d'analyse qualitative et quantitative des acides nucléiques, notamment ADN, des différentes espèces microbiologiques constituant le levain selon l'invention, notamment par des techniques de réaction en chaîne par polymérase (PCR).

On utilise un tel levain pour la préparation de spécialités fermentées. L'utilisation d'un tel levain permet de conférer à la spécialité fermentée des caractéristiques organoleptiques, notamment de goût et de texture proches de celles formées à partir de lait cru. Une telle utilisation permet en outre un développement harmonieux du produit lors de son affinage. En outre, l'utilisation d'un tel levain permet d'augmenter la date limite d'utilisation optimale (D.L.U.O.) du produit fabriqué à partir du levain en apportant au produit une composition microbiologique diversifiée adaptée pour prévenir, par un effet barrière, d'éventuelles contaminations ultérieures à la préparation du produit. Une telle composition microbiologique diversifiée, apportée dans le procédé de fabrication d'un produit laitier fermenté, est adaptée pour prévenir le développement d'une espèce microbiologique majoritaire, voire unique, susceptible de détourner au profit de son développement la totalité du substrat disponible. En particulier, l'utilisation d'un tel levain permet d'éviter la production désagréable d'ammoniac en privilégiant les flores d'affinage saines.

L'utilisation d'un tel levain est adaptée pour permettre une augmentation du taux d'eau liée de la spécialité fermentée par rapport à l'eau libre de celle-ci, conférant à ladite spécialité, une stabilité dans le temps, une texture avantageuse et, lors de l'affinage en cave ou sous emballage, des pertes d'eau maîtrisées et plus faibles. De telles pertes d'eau faibles et maîtrisées engendrent des augmentations du rendement de production de ladite spécialité et un intérêt économique.

L'invention concerne en outre l'utilisation d'un levain selon pour la préparation d'un produit laitier fermenté dans laquelle on réalise une fermentation d'un lait cru. En particulier on utilise un tel levain dans l'industrie fromagère pour la préparation d'un produit laitier fermenté de type « à pâte cuite », d'un produit laitier fermenté de type « à pâte pressée », d'un produit laitier fermenté de type « à pâtes molles », d'un produit laitier fermenté de type « à pâte lactique », d'un produit laitier fermenté de type « à pâte persillée », des yaourts, des laits fermentés et du beurre.

On utilise un tel levain pour la préparation d'un produit laitier fermenté dans lequel on réalise une fermentation d'un lait, choisi dans le groupe formé des laits crus, des laits crus ayant subit un traitement thermique -notamment des laits pasteurisés et des laits thermisés-, des laits crus ayant subit un traitement athermique -notamment des laits micro-filtrés-, des laits reconstitués et des laits standardisés.

En particulier, l'utilisation d'un tel levain pour la préparation d'un produit laitier fermenté à partir de lait pasteurisé, thermisé ou micro-filtré, permet avantageusement de produire un produit laitier fermenté au lait pasteurisé, thermisé ou micro-filtré présentant des propriétés organoleptiques proches des propriétés organoleptiques d'un produit laitier au lait cru.

L'utilisation d'un levain obtenu lors du traitement de stabilisation, permet en outre d'augmenter la reproductibilité des procédés de préparation des produits laitiers fermentés par stabilisation de la composition microbiologique. En outre, l'utilisation d'un tel levain, de composition complexe, permet de prévenir le produit d'une évolution de sa maturation liée à une espèce microbiologique unique. L'utilisation d'un tel levain permet en outre d'augmenter la quantité d'eau liée au produit laitier fermenté et d'augmenter ainsi le rendement de production du produit.

L'utilisation d'un levain biodiversifié est en particulier adaptée pour s'opposer au développement et/ou freiner la croissance de bactéries d'hygiène et/ou pathogènes, dans un milieu de fabrication d'un produit laitier fermenté, dans le produit laitier fermenté lui-même lors de sa conservation évolutive. Un tel effet protecteur est en outre adapté pour prévenir de développement de bactéries d'hygiène et/ou pathogènes dans la flore intestinale au cours de la digestion du produit laitier fermenté selon l'invention.

L'invention concerne également un procédé de préparation d'un levain, caractérisé en combinaison par tout ou partie des caractéristiques mentionnées ci-dessus ou ci-après.

D'autres buts, caractéristiques et avantages de l'invention apparaîtront à la lecture de la description suivante qui se réfère aux figures annexées représentant des modes de réalisation préférentiels de l'invention, donnés uniquement à titre d'exemples non limitatifs, et dans lesquelles :
- la figure 1 est un schéma synoptique illustrant un procédé selon l'invention,
- la figure 2 est une représentation graphique de l'évolution temporelle du pH d'un milieu fermentation formé d'un lait cru comprenant de l'ordre de 2 10⁵ micro-organismes/mL d'une première étape de fermentation stabilisante à 27°C d'un procédé selon l'invention,
- la figure 3 est une représentation graphique de l'évolution temporelle du pH d'un milieu fermentation formé d'un lait cru comprenant de l'ordre de 1,1 10⁴ micro-organismes/mL d'une première étape de fermentation stabilisante à 27°C d'un procédé selon l'invention,
- La figure 4 est une représentation en bâtonnets de la composition microbienne des milieux de fermentation successifs d'un traitement de stabilisation d'un lait cru comprenant de l'ordre de 2,0 10⁵ micro-organismes/mL.
- La figure 5 est une représentation en bâtonnets de la composition microbienne des milieux de fermentation successifs d'un traitement de stabilisation d'un lait cru comprenant de l'ordre de 1,1 10⁴ micro-organismes/mL.
- la figure 6 est une représentation en bâtonnets de la composition microbienne des milieux de fermentation successifs d'un traitement de stabilisation d'un lait cru comprenant de l'ordre de 6,5 10³ micro-organismes/mL.
- la figure 7 est une représentation graphique de l'évolution temporelle de la composition microbienne d'un milieu de fermentation d'une troisième étape de fermentation stabilisante d'un traitement de stabilisation d'un lait cru comprenant de l'ordre de 2,0 10⁵ micro-organismes/mL selon l'invention,
- la figure 8 est une représentation graphique de l'évolution temporelle du pourcentage des flores d'altération dans un milieu de fermentation d'une troisième étape de fermentation stabilisante d'un traitement de stabilisation selon l'invention d'un lait cru comprenant de l'ordre de 2,0 10⁵ micro-organismes/mL,
- la figure 9 est une représentation graphique d'un profil d'analyse électrophorétique des constituants microbiologiques d'un levain obtenu par un procédé selon l'invention,
- La figure 10 est une représentation graphique d'un profil d'analyse électrophorétique des constituants microbiologiques d'un levain obtenu à partir de co-culture.
- La figure 11 est une représentation graphique d'un profil d'analyse électrophorétique des constituants microbiologiques d'un lait cru.

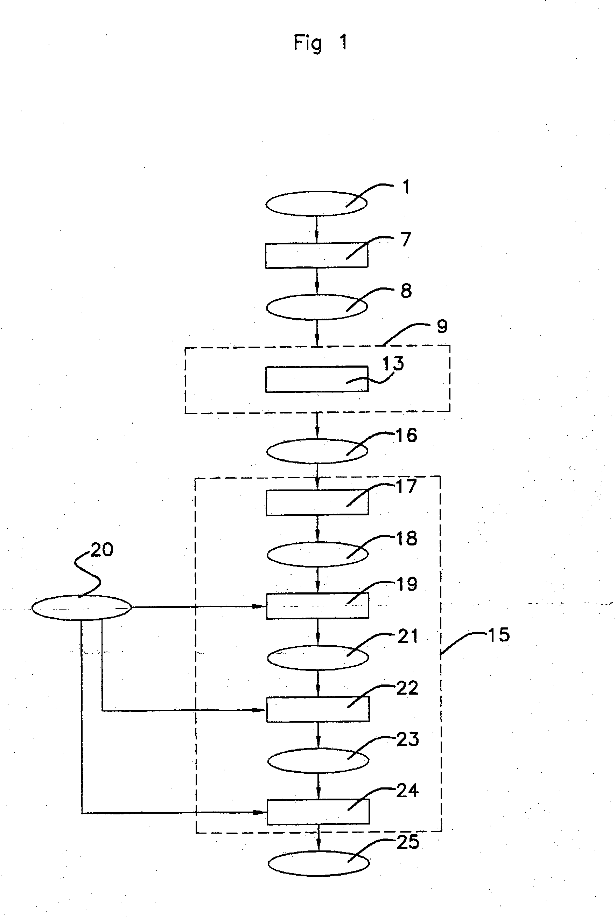

Un mode de réalisation d'un procédé de préparation d'un levain selon l'invention est illustré par le schéma synoptique de la figure 1. On réalise un prélèvement 7 d'une quantité de lait cru dans une exploitation laitière d'un groupe 1 d'exploitations laitières représentatives d'un terroir. L'ensemble des prélèvements 7 des laits crus constitue une collection 8 de prélèvements de laits crus. On réalise ce prélèvement 7 après la traite, notamment dans un délai suivant la traite inférieur à 12 h, et on soumet cette quantité de lait prélevé directement à un traitement de refroidissement adapté pour préserver durablement la composition de la flore vivante constitutive du lait cru. En particulier on réalise le traitement de refroidissement d'un volume du lait cru à une température de +4°C. Le volume de lait cru soumis au traitement de refroidissement est adapté pour permettre un refroidissement rapide, notamment en moins de 1 h, de ce volume de lait cru. Le volume de lait cru soumis au traitement de refroidissement est en particulier de l'ordre de 1 L.

On réalise ensuite une étape 9 d'évaluation des différents laits crus de la collection 8 de laits crus de façon à identifier et à sélectionner les laits crus susceptibles de permettre la préparation d'un levain selon l'invention. L'étape 9 d'évaluation des laits crus de la collection 8 comprend au moins une étape 13 de sélection des laits crus dans laquelle on réalise, à au moins une température de sélection, une fermentation lactique acidifiante du lait cru et à l'issue de laquelle on retient les laits dont le pH du milieu de fermentation a atteint une valeur de 5,5 en plus de 15 h, et une valeur minimum de 5,0 en moins de 48 h. En particulier on réalise en parallèle une étape 13 de sélection d'un lait cru à la température de 27°C et/ou à la température de 40°C, et on retient le(s) lait(s) cru(s) dont le pH du milieu de fermentation lactique acidifiante à 27°C et/ou à 40°C a atteint une valeur de 5,5 en plus de 15 h, et une valeur minimum de 5,0 en moins de 48 h à 27°C et à 40°C. On évalue ainsi le potentiel des laits crus de la collection 8 de laits crus à induire une diminution du pH d'un milieu de fermentation, par fermentation lactique acidifiante, qui soit lente et néanmoins stabilisante. On réalise cette étape 13 de sélection de lait cru dans un réacteur adapté pour permettre de mesurer, notamment en continu, le pH du milieu de fermentation. En particulier, on réalise cette étape 13 de sélection dans un fermenteur permettant de maintenir la température du milieu de fermentation à une température présélectionnée. On réalise cette étape 13 de sélection sous agitation dudit milieu de fermentation. Cependant, cette agitation adaptée pour homogénéiser le milieu de fermentation n'est pas adaptée pour permettre une aération du milieu de fermentation. Ainsi, la fermentation lactique acidifiante se produit sensiblement dans des conditions d'anaérobiose.

On soumet, lors d'une première étape 17 de fermentation stabilisante d'un traitement 15 de stabilisation, un milieu de fermentation formé d'un lait 16 cru présélectionné, à une fermentation lactique acidifiante, à une température de 27°C, et on interrompt ladite fermentation lactique acidifiante lorsque le pH du milieu de fermentation atteint la valeur de 5,2 en plus de 12 h en moins de 24 h. On interrompt ladite fermentation lactique acidifiante par refroidissement du milieu de fermentation à une température de l'ordre de +4°C. On obtient, à l'issue de cette première étape 17 de fermentation stabilisante, un milieu de fermentation formé d'un lait 18 fermenté dont le pH est de l'ordre de pH 5,2, dont la température est de l'ordre de +4°C, et dont la flore microbiologique est enrichie en regard de la flore du lait cru de départ.

Dans une deuxième étape 19 ultérieure de fermentation stabilisante, on prépare un milieu de fermentation d'une deuxième étape de fermentation stabilisante, formé du mélange d'une quantité du lait 18 fermenté et d'une quantité d'un milieu 20 de culture liquide, de façon que la proportion volumique de lait 18 fermenté dans le milieu de fermentation d'une deuxième étape de fermentation stabilisante soit de l'ordre de 2%. On réalise une fermentation lactique acidifiante à 27°C du milieu de fermentation de ladite deuxième étape de fermentation stabilisante et on interrompt la fermentation lactique acidifiante lorsque le pH du milieu de fermentation de la deuxième étape de fermentation stabilisante atteint la valeur de 5,2. On interrompt ladite fermentation lactique acidifiante par refroidissement à +4°C du milieu de fermentation à pH 5,2 de la deuxième étape de fermentation stabilisante.

Dans ce mode de réalisation d'un procédé selon l'invention, on réalise une pluralité d'étapes successives de fermentation stabilisante, et le milieu de fermentation de chaque étape de fermentation stabilisante est formé d'une quantité d'un milieu 20 de culture liquide et d'une quantité d'un milieu de fermentation à pH 5,2, issu d'une étape préalable de fermentation stabilisante.

Ainsi, lors d'étapes successives 22 et 24 ultérieures de fermentation stabilisante, on prépare un milieu de fermentation pour une étape ultérieure de fermentation stabilisante d'un traitement 15 de stabilisation, formé du mélange d'une quantité d'un milieu 21, respectivement 23 de fermentation à pH 5,2, avec une quantité d'un milieu 20 de culture liquide, de façon que la proportion volumique du milieu de fermentation 21, 23 à pH 5,2 dans le milieu de fermentation d'une étape ultérieure de fermentation stabilisante soit de l'ordre de 2%. On réalise une fermentation lactique acidifiante à 27°C du milieu de fermentation d'une étape ultérieure de fermentation stabilisante et on interrompt la fermentation lactique acidifiante lorsque le pH du milieu de fermentation de l'étape ultérieure de fermentation stabilisante atteint la valeur de 5,2. On interrompt ladite fermentation lactique acidifiante par refroidissement du milieu de fermentation de l'étape ultérieure de fermentation stabilisante à une température sensiblement de l'ordre de +4°C.

Le milieu de fermentation obtenu à l'issue de l'étape 24 de fermentation stabilisante est un levain 25, présentant une concentration élevée, notamment de l'ordre de 2 10⁹ micro-organismes/mL et comprenant l'essentiel des espèces microbiologiques constitutives de la flore utile du lait 16 cru sélectionné.

Dans un deuxième mode de réalisation non représenté d'un procédé selon l'invention, il est possible de réaliser, après l'étape 24 de fermentation stabilisante, une pluralité -notamment une ou deux- étapes ultérieures additionnelles de fermentation stabilisante d'un traitement 15 de stabilisation en réalisant une fermentation lactique acidifiante à 27°C d'un milieu de fermentation formé d'une quantité d'un milieu de fermentation stabilisante à pH 5,2 d'une étape antérieure de fermentation stabilisante du traitement 15 de stabilisation, et d'une quantité d'un milieu 20 de culture et en interrompant ladite fermentation lactique acidifiante lorsque le pH du milieu de fermentation atteint la valeur de 5,2. On interrompt ladite fermentation lactique acidifiante par refroidissement du milieu de fermentation de l'étape de fermentation stabilisante à une température sensiblement de l'ordre de +4°C.

Dans un troisième mode de réalisation non représenté d'un procédé selon l'invention, on utilise pour la préparation des milieux de fermentation des étapes de fermentation stabilisante successives d'un traitement 15 de stabilisation, des milieux 20 de culture de compositions différentes que l'on mélange avec une quantité d'un milieu de fermentation issu d'une étape précédente de fermentation stabilisante.

EXEMPLE 1 : Préparation d'un levain à partir d'un lait cru comprenant de l'ordre de 2 10⁵ micro-organismes vivants/mL.

On choisit un lait cru de vache dans une exploitation laitière faisant partie d'un terroir, préalablement sélectionnée en regard des conditions d'élevage du bétail, notamment dans une exploitation laitière préconisant un mode de pâturage permanent. On prélève 1 L de ce lait que l'on conserve à +4°C jusqu'au traitement de stabilisation. Ce lait présente une numération de FMAR (Flore Mésophile Aérobie Revivifiable) élevée, de l'ordre de 2 10⁵ micro-organismes/mL. On place ce lait sous agitation, à une température de 27°C dans un fermenteur adapté pour permettre une fermentation lactique du lait cru. En particulier, on réalise cette fermentation lactique dans un fermenteur adapté pour permettre de mesurer le pH du milieu de fermentation.

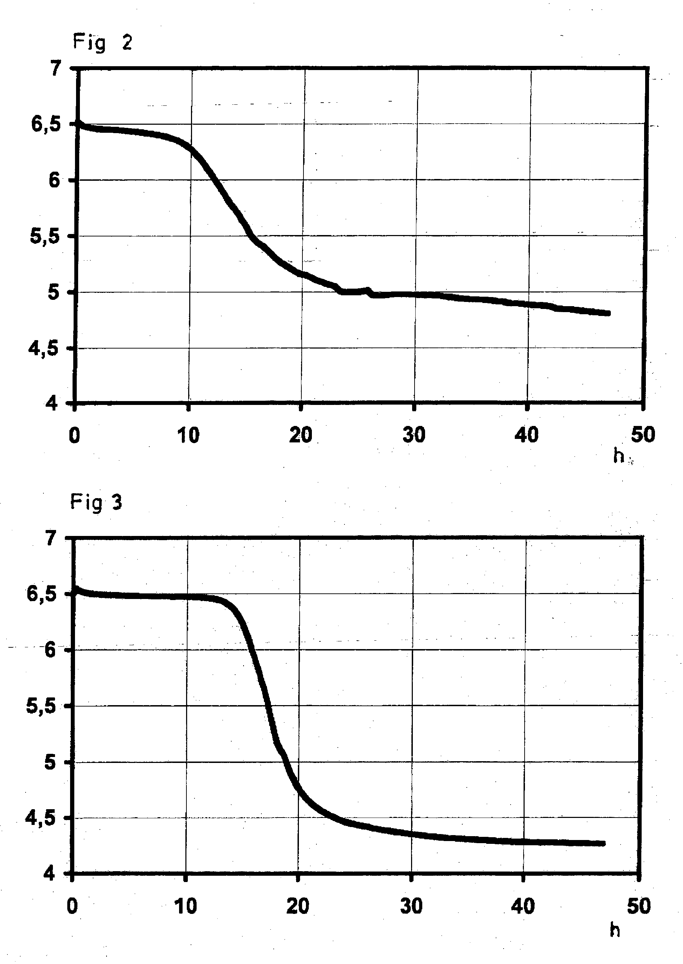

La figure 2 illustre la variation du pH d'un milieu de fermentation, formé d'un lait cru comprenant de l'ordre de 2 10⁵ micro-organismes/mL, d'une première étape de fermentation stabilisante d'un traitement de stabilisation à 27°C selon l'invention. La figure 2 représente ladite variation du pH du milieu de fermentation de ladite première étape de fermentation stabilisante à 27°C lorsque la fermentation lactique acidifiante n'est pas interrompue lorsque le pH dudit milieu de fermentation atteint une valeur de l'ordre de 5,2. La durée de la phase de latence de la courbe d'acidification, définie comme la partie précoce de la cinétique au cours de laquelle la valeur du pH est sensiblement invariante, est de l'ordre de 10 h. Le pH du milieu de fermentation atteint la valeur de 6,0 après une durée de fermentation lactique acidifiante de 12 h, la valeur de 5,2 après 18 h et la valeur de 4,9 après 24 h. Le pH du milieu de fermentation se stabilise à une valeur de l'ordre de 4,4 après environ 30 h de fermentation. Ainsi, pour la mise en oeuvre d'un procédé selon l'invention, le milieu de fermentation de la première étape de fermentation stabilisante est un lait cru et la fermentation lactique acidifiante du milieu de fermentation est avantageusement interrompue lorsque le pH du milieu de fermentation atteint 5,2, notamment dans cet exemple au bout de 18 h.

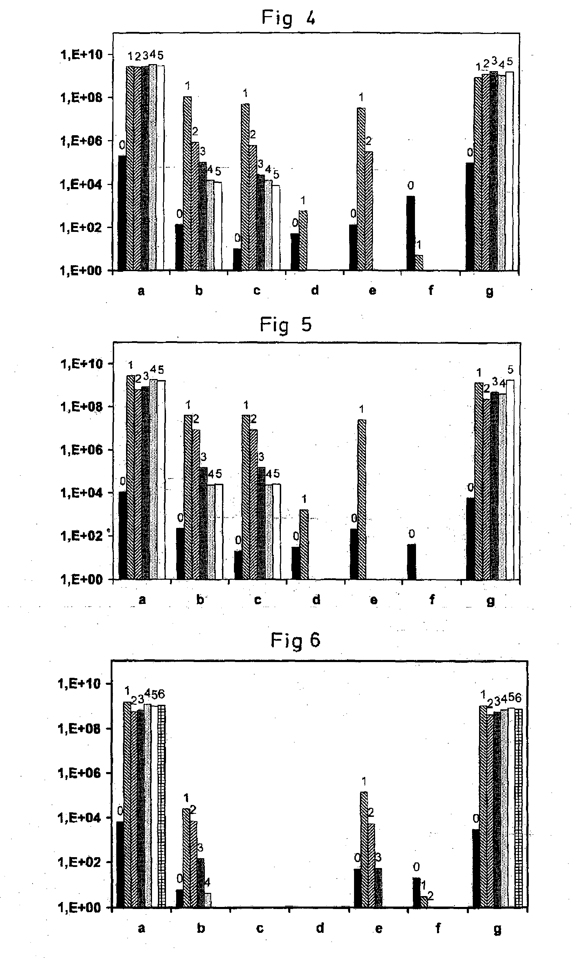

La figure 4 représente, à titre d'exemple non limitatif, l'évolution de la composition de la flore (FMAR) mésophile aérobie revivifiable (a), de la flore coliforme (b), d'*Escherichia coli* (c), des staphylocoques (d), des levures (e), des moisissures (f) et de la flore utile (g) du milieu de fermentation au cours des étapes successives d'un traitement de stabilisation d'un lait cru comprenant de l'ordre de 2 10⁵ micro-organismes/mL selon l'invention. L'analyse correspondant à la série 0 de la figure 4 (bâtonnets pleins) représente la numération microbiologique du lait cru de départ. Les analyses correspondant aux séries 1, 2, 3, 4, et 5 de la figure 4 représentent respectivement les numérations microbiologiques du milieu de fermentation à pH 5,2 respectivement de la première, de la deuxième, de la troisième, de la quatrième, et de la cinquième étape de fermentation stabilisante d'un traitement de stabilisation selon l'invention.

Les valeurs numériques correspondant à ces numérations sont données dans le tableau 1 ci-dessous. La composition microbiologique de la flore utile (g) est détaillée dans le tableau 1 et regroupe l'ensemble des streptocoques (h), des lactocoques (i), des lactobacilles (j) et des leuconostoc (k).

**Tableau 1**

| | **a** | **b** | **c** | **d** | **e** | **f** | **g** | | | |
|---|---|---|---|---|---|---|---|---|---|---|
| | | | | | | | **h** | **I** | **j** | **k** |
| **0** | 2,04E+5 | 1,3E+2 | 1,0E+1 | 5,0E+1 | 1,3E+2 | 2,8E+3 | 8,4E+3 | 4,08E+4 | 4,4E+3 | 4,24E+4 |
| **1** | 2,70E+9 | 1,06E+8 | 4,90E+7 | 5,66E+2 | 3,10E+7 | 5,0E+0 | 2,49E+8 | 2,6E+8 | 3,06E+8 | 4,52E+6 |
| **2** | 2.44E+9 | 8,20E+5 | 5,90E+5 | nd | 3,0E+5 | nd | 4,30E+8 | 5,5E+8 | 2,06E+8 | 8,5E+6 |
| **3** | 2,76E+9 | 1,03E+5 | 2,65E+4 | nd | nd | nd | 7,0E+8 | 7,8E+8 | 1,02E+8 | 3,0E+7 |
| **4** | 3,25E+9 | 1,50E+4 | 1,45E+4 | nd | nd | nd | 8,4E+8 | 1,08E+8 | 1,08E+8 | 1,44E+7 |
| **5** | 2.85E+9 | 1,20E+4 | 8.50E+3 | nd | nd | nd | 9,5E+8 | 4,5E+8 | 9,6E+7 | 1,13E+7 |

| | | | | | | | | | | |
|---|---|---|---|---|---|---|---|---|---|---|
| nd = non détectable | | | | | | | | | | |

Lors de la première étape de fermentation stabilisante, la numération de la FMAR (a) passe d'une valeur initiale (série 0) dans le lait cru de 2,04 10⁵ micro-organismes/mL, à une valeur finale dans les milieux dé fermentation à pH 5,2 (séries 1, 2, 3, 4 et 5) successifs de l'ordre de 2,80 10⁹ micro-organismes/mL. La numération de la flore utile (g) passe d'une valeur initiale (série 0) dans le lait cru de 9,6 10⁵ micro-organismes/mL, à une valeur finale dans les milieux de fermentation à pH 5,2 (séries 1, 2, 3, 4 et 5) successifs qui est stable et de l'ordre de 1,3 10⁹ micro-organismes/mL.

En outre, la numération de la flore coliforme (c) augmente significativement d'une valeur initiale (série 0) dans le lait cru de 140 micro-organismes/mL, à une valeur dans le milieu de fermentation à pH 5,2 (série 1) de la première étape de fermentation stabilisante de 1,06 10⁸ micro-organismes/mL, puis décroît dans les milieux de fermentation des étapes ultérieures de fermentation stabilisante successives (séries 2, 3, 4 et 5) jusqu'à une valeur de l'ordre de 1,20 10⁴ micro-organisines/mL. Ainsi, la proportion de bactéries coliformes par rapport à la FMAR passe d'une valeur de l'ordre de 0,06% dans le lait cru, à une valeur de l'ordre de 0,0004% dans le levain obtenu après la cinquième étape de fermentation stabilisante traduisant un épuisement du levain en flores coliformes.

Une observation similaire est faite vis-à-vis des bactéries *Escherichia coli* dont la proportion par rapport à la FMAR est de 1 bactérie E.coli pour 2 10⁴ FMAR dans le lait cru et de 1 bactérie E.coli pour 3 10⁶ FMAR dans le milieu de fermentation de la cinquième étape de fermentation stabilisante.

On observe en outre sur la figure 4 et dans le tableau 1 que les staphylocoques (d), les levures (e) et les moisissures (f) disparaissent totalement des milieux de fermentation successifs dès la troisième étape (série 4) de fermentation stabilisante d'un traitement de stabilisation selon l'invention.

En outre, les inventeurs ont observé que les cinétiques d'acidification des milieux de fermentation des étapes de fermentation stabilisante d'un traitement de stabilisation selon l'invention sont similaires à partir de la deuxième étape de fermentation stabilisante. Ces cinétiques présentent un temps de latence de l'ordre de 150 min, atteignent le pH de 6,0 entre 230 min et 260 min, le pH de 5,2 entre 290 min et 320 min et se stabilisent à un pH de l'ordre de pH 4,5 au bout de 400 min.

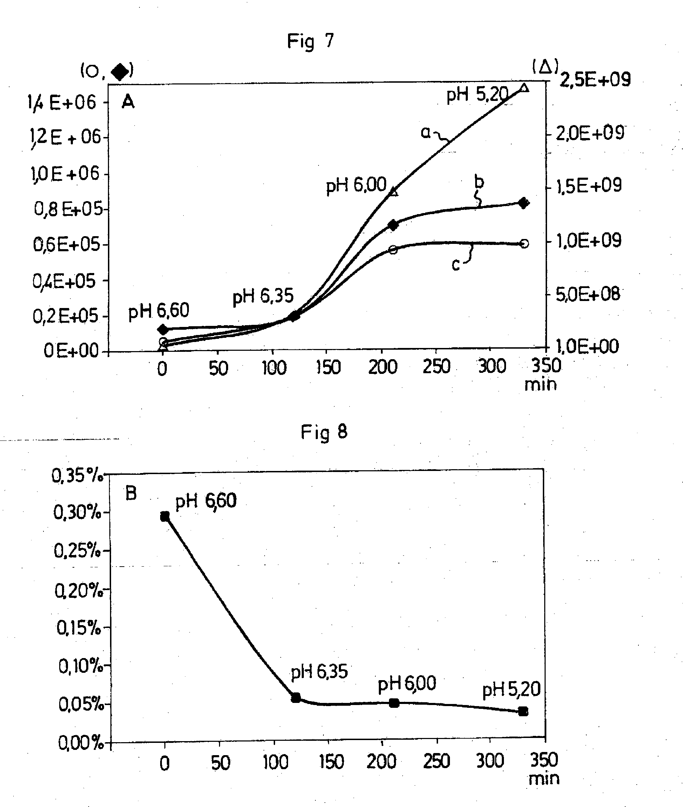

La figure 7 représente l'évolution des numérations bactériennes au cours de la troisième étape de fermentation stabilisante du traitement de stabilisation d'un lait cru comprenant de l'ordre de 2 10⁵ micro-organismes/mL selon l'invention, conduite à la température de 27°C et interrompue après 330 min lorsque le pH du milieu de fermentation a atteint la valeur de 5,2. La courbe (a) représente l'évolution de la flore totale (FMAR), la courbe (b) représente l'évolution de la flore formée des coliformes, et la courbe (c) représente l'évolution de la flore formée d'*Escherichia coli.* On observe que la flore coliforme et la flore formée d'*Escherichia coli* présentent un taux de croissance qui diminue à partir de 200 min (pH 6,0) de fermentation lactique, alors que la flore totale (FMAR) présente un taux de croissance qui n'est pas sensiblement ralenti jusqu'à pH 5,2.

La figure 8 représente l'évolution de la numération de la flore coliforme, exprimée en pourcentage de la flore totale (FMAR), dans le milieu de fermentation d'une troisième étape de fermentation stabilisante d'un procédé selon l'invention. On note que la proportion de la flore coliforme par rapport à la flore totale du milieu de fermentation lors de l'ensemencement est de 0,29 % et diminue jusqu'à la valeur de 0,06% après 120 min d'incubation (pH 6,35) et s'équilibre à la valeur de 0,03% après 330 min d'incubation (pH 5,20).

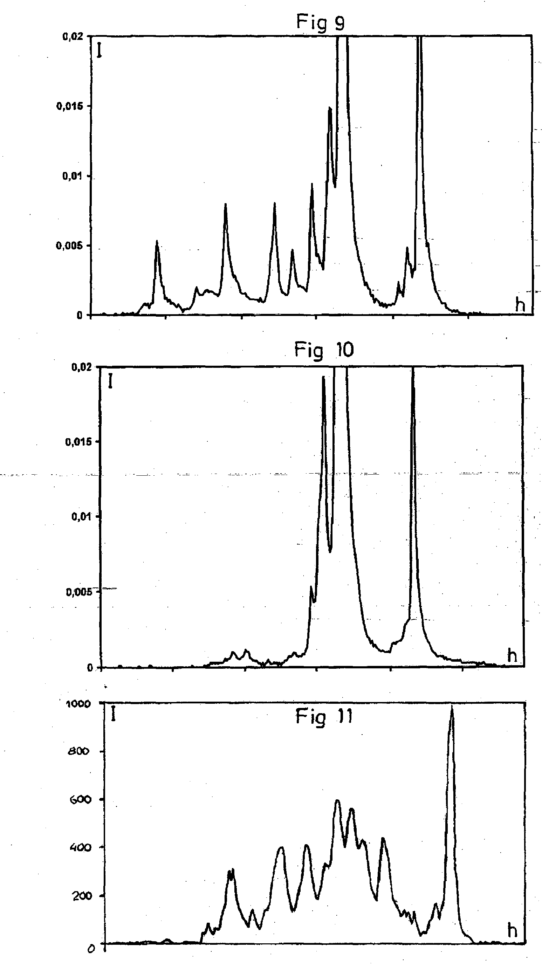

La figure 9 représente une empreinte moléculaire montrant la biodiversité microbiologique du levain obtenu par un procédé selon l'invention à partir du lait cru de l'exemple 1 comprenant de l'ordre de 2 10⁵ micro-organismes/mL. Le profil de la figure 9 montre deux pics majeurs correspondant aux souches microbiologiques lactofermentaires majeures du levain. En outre, le profile montre huit pics d'intensité intermédiaire caractérisant au moins huit espèces microbiologiques différentes dans le levain selon l'invention. Enfin le profil révèle une multiplicité de signaux de faible intensité mais caractérisant une biodiversité importante du levain selon l'invention élaboré à partir de lait cru.

En outre, le profil d'analyse, à titre de contrôle, d'un lait cru tel qu'utilisé pour la préparation d'un levain selon l'invention, présente un pic majeur correspondant à une souche microbiologique lactofermentaire du lait cru, huit pics d'intensité intermédiaire, et une multiplicité de signaux de faible intensité caractéristique de la biodiversité du lait cru. Ce contrôle montre qu'un procédé de préparation d'un levain selon l'invention permet de conserver l'essentiel de la biodiversité du lait cru à l'origine du levain préparé.

La figure 10 représente à titre de contrôle comparatif un profil d'analyse d'un levain obtenu à partir de co-culture montrant deux pics majeurs correspondant aux souches microbiologiques lactofermentaires, et deux pics d'intensité intermédiaire. En outre, le profil ne révèle pas de signaux de faible intensité révélateurs d'une biodiversité importante du levain.

La figure 11 représente une empreinte ADN d'un lait cru, donné à titre de comparaison des compositions respectives d'un lait cru et d'un levain obtenu par un procédé selon l'invention. On observe que l'empreinte ADN du levain selon l'invention est comparable à celle du lait cru de référence, révélant ainsi la quasi-conservation de la biodiversité du levain par rapport au lait cru.

EXEMPLE 2 : Préparation d'un levain à partir d'un lait cru comprenant de l'ordre de 1 10⁴ micro-organismes/mL.

On prélève 1 L d'un lait cru de vache présentant une numération de FMAR (Flore Mésophile Aérobie Revivifiable) faible de l'ordre de 1 10⁴ micro-organismes/mL. On traite ce lait cru dans des conditions semblables à celles décrites dans l'exemple 1.

La figure 3 illustre un exemple de la variation du pH d'un milieu de fermentation, formé d'un lait cru comprenant de l'ordre de 1,1 10⁴ micro-organismes/mL, d'une première étape de fermentation stabilisante d'un traitement de stabilisation à 27°C selon l'invention. La figure 3 représente ladite variation du pH du milieu de fermentation de ladite première étape de fermentation stabilisante à 27°C lorsque la fermentation lactique acidifiante n'est pas interrompue lorsque le pH dudit milieu de fermentation atteint une valeur de l'ordre de 5,2. La durée de la phase de latence, définie comme la partie précoce de la cinétique au cours de laquelle la valeur du pH est sensiblement invariante, est de l'ordre de 15 h. Le pH du milieu de fermentation atteint la valeur de 6,0 après une durée de fermentation lactique acidifiante de 16 h, la valeur de 5,2 après 18 h et la valeur de 4,9 après 19 h. Le pH du milieu de fermentation se stabilise à une valeur de l'ordre de 4,3 en environ 30 h. Ainsi, pour la mise en oeuvre d'un procédé selon l'invention, le milieu de fermentation de la première étape de fermentation stabilisante est un lait cru et la fermentation lactique acidifiante du milieu de fermentation serait avantageusement interrompue lorsque le pH du milieu de fermentation atteint 5,2, notamment au bout de 18 h.

La figure 5 représente l'évolution de la composition de la flore (FMAR) mésophile aérobie revivifiable (a), de la flore coliforme (b), d'*Escherichia coli* (c), des staphylocoques (d), des levures (e), des moisissures (f) et de la flore utile (g) du milieu de fermentation au cours des étapes successives d'un traitement de stabilisation d'un lait cru comprenant de l'ordre de 1,1 10⁴ micro-organismes/mL. L'analyse correspondant à la série 0 de la figure 5 (bâtonnets pleins) représente la numération du lait cru de départ. Les analyses correspondant aux séries 1, 2, 3, 4, et 5 de la figure 5 représentent respectivement les numérations du milieu de fermentation à pH 5,2 respectivement de la première, de la deuxième, de la troisième, de la quatrième, et de la cinquième étape de fermentation stabilisante du traitement de stabilisation.

Les valeurs numériques correspondant à ces numérations sont données dans le tableau 2 ci-dessous. La composition microbiologique de la flore utile (g) détaillée dans le tableau 2 regroupe l'ensemble des streptocoques (h), des lactocoques (i), des lactobacilles (j) et des leuconostoc (k). Dans le tableau 2, nd signifie non détectable.

**Tableau 2**

| | **a** | **b** | **c** | **d** | **e** | **f** | **g** | | | |
|---|---|---|---|---|---|---|---|---|---|---|
| | | | | | | | **h** | **i** | **j** | **k** |
| **0** | 1,1E+4 | 2,2E+2 | 2,0E+1 | 3,0E+1 | 2,0E+2 | 4,0E+1 | 4,1E+3 | 1,3E+3 | 4,0E+2 | 1,2E+2 |
| **1** | 2,72E+9- | 3,84E+7 | 3,84E+7 | 1,6E+3 | 2,4E+7 | nd | 2,0E+8 | 2,32E+8 | 8,0E+8 | 9,6E+7 |
| **2** | 6,3E+8 | 8,4E+6 | 8,4E+6 | nd | nd | nd | 4,5E+7 | 3,5E+7 | 1,53E+8 | 3,0E+6 |
| **3** | 8,4E+8 | 1,5E+5 | 1,5E+5 | nd | nd | nd | 1,16E+8 | 1,16E+8 | 2,5E+8 | 1,1E+6 |
| **4** | 1,76E+9 | 2,3E+4 | 2,3E+4 | nd | nd | nd | 1,08E+8 | 1,88E+8 | 1,16E+8 | 7,4E+6 |
| **5** | 1,6E+9 | 2,5E+4 | 2,5E+4 | nd | nd | nd | 4,2E+8 | 1,08E+9 | 1,52E+8 | 1,3E+8 |

| | | | | | | | | | | |
|---|---|---|---|---|---|---|---|---|---|---|
| nd = non détectable | | | | | | | | | | |

Lors de la première étape de fermentation stabilisante, la numération de la FMAR (a) passe d'une valeur initiale (série 0) dans le lait cru de 1,1 10⁴ micro-organismes/mL, à une valeur finale dans les milieux de fermentation à pH 5,2 (séries 1, 2, 3, 4 et 5) successifs de l'ordre de 1,2 10⁹ micro-organismes/mL. La numération de la flore utile (g) passe d'une valeur initiale (série 0) dans le lait cru de 5,9 10³ micro-organismes/mL, à une valeur finale moyenne dans les milieux de fermentation à pH 5,2 (séries 1, 2, 3, 4 et 5) successifs qui est stable et de l'ordre de 1,06 10⁹ micro-organismes/mL.

En outre, la numération de la flore coliforme (c) augmente significativement d'une valeur initiale (série 0) dans le lait cru de 220 micro-organismes/mL, à une valeur dans le milieu de fermentation à pH 5,2 (série 1) de la première étape de fermentation stabilisante de 3,84 10⁷ micro-organismes/mL, puis décroît dans les milieux de fermentation des étapes ultérieures de fermentation stabilisante successives (séries 2, 3, 4 et 5) jusqu'à une valeur de l'ordre de 2,50 10⁴ micro-organismes/mL. Ainsi, la proportion de bactéries coliformes par rapport à la FMAR passe d'une valeur de l'ordre de 2% dans le lait cru, à une valeur de l'ordre de 0,001.5% dans le levain obtenu après la cinquième étape de fermentation stabilisante traduisant ainsi un appauvrissement de la flore colifonne du levain.

On observe également que les bactéries *Escherichia coli* dont la proportion par rapport à la FMAR est de 1 bactérie *Escherichia coli* pour 550 bactéries de la flore totale (FMAR) dans le lait cru et de 1 bactérie *Escherichia coli* pour 6,4 104 FMAR dans le milieu de fermentation de la cinquième étape de fermentation stabilisante traduisant ainsi un appauvrissement des bactéries *Escherichia coli* du levain.

On observe en outre sur la figure 5 et dans le tableau 2 que les staphylocoques (d), les levures (e) et les moisissures (f) disparaissent totalement des milieux de fermentation successifs dès la deuxième étape (série 3) de fermentation stabilisante d'un traitement de stabilisation selon l'invention.

En outre, les inventeurs ont observé que les cinétiques d'acidification des milieux de fermentation des étapes de fermentation stabilisante d'un traitement de stabilisation selon l'invention sont similaires à partir de la deuxième étape de fermentation stabilisante. Ces cinétiques présentent un temps de latence de l'ordre de 150 min, atteignent le pH de 6,0 entre 210 min et 250 min, le pH de 5,2 entre 290 min et 320 min et se stabilisent à un pH de l'ordre de pH 4,5 au bout de 400 min.

EXEMPLE 3 : Préparation d'un levain à partir d'un lait cru comprenant de l'ordre de 6,5 10³ micro-organismes/mL.

On prélève 1 L d'un lait cru de vache présentant une numération de FMAR (Flore Mésophile Aérobie Revivifiable) faible de l'ordre de 6,5 10³ micro-organismes/mL. On traite ce lait cru dans des conditions semblables à celles décrites dans l'exemple 1.

La figure 6 représente l'évolution de la composition de la flore (FMAR) mésophile aérobie revivifiable (a), de la flore coliforme (b), d'*Escherichia coli* (c), des staphylocoques (d), des levures (e), des moisissures (f) et de la flore utile (g) du milieu de fermentation au cours des étapes successives d'un traitement de stabilisation d'un lait cru ultra-propre comprenant de l'ordre de 6,5 10³ micro-organismes/mL. L'analyse correspondant à la série 0 de la figure 6 (bâtonnets pleins) représente la numération du lait cru de départ. Les analyses correspondant aux séries 1, 2, 3, 4, 5 et 6 de la figure 6 représentent respectivement les numérations du milieu de fermentation à pH 5,2 respectivement de la première, de la deuxième, de la troisième, de la quatrième, de la cinquième et de la sixième étape de fermentation stabilisante du traitement de stabilisation:

Les valeurs numériques correspondant à ces numérations sont données dans le tableau 3 ci-dessous. La composition microbiologique de la flore utile (g) détaillée dans le tableau 3 regroupe l'ensemble des streptocoques (h), des lactocoques (i), des lactobacilles (j) et des leuconostoc (k). Dans le tableau 3, nd signifie non détectable.

**Tableau 3**

| | **a** | **b** | **c** | **d** | **e** | **f** | **g** | | | |
|---|---|---|---|---|---|---|---|---|---|---|
| | | | | | | | **h** | **i** | **j** | **k** |
| **0** | 6,5E+3 | -6,0E+0 | nd | nd | 5,0E+1 | 2,0E+1 | 2,05E+3 | 7,5E+2 | 2,5E+2 | 1E+1 |
| **1** | 1,55E+9 | 2,46E+4 | nd | nd | 1,4E+5 | 3E+0 | 3,5E+8 | 1,53E+8 | 5,92E+8 | 1,54E+6 |
| **2** | 5,5E+8 | 7,2E+3 | nd | nd | 5,4E+3 | 1E+0 | 2,SE+8 | 7,5E+7 | 9,7E+7 | 5,3E+6 |
| **3** | 6,5E+8 | 1,45E±2 | nd | nd | 5,3E+1 | nd | 1,9E+8 | 2,84E+8 | 7,5E+7 | 4,3E+6 |
| **4** | 1,2E+9 | 4E+0. | nd | nd | nd | nd | 2,2E+8 | 3,4E+8 | 1,4E+8 | 2,1E+6 |
| **5** | 9,95E+8 | nd | nd | nd | nd | nd | 2,7E+8 | 4,5E+8 | 1,2E+8 | 4,7E+6 |
| **6** | 1,1E+9 | nd | nd | nd | nd | nd | 3,5E+8 | 3,2E+8 | 9,8E+7 | 5,5E+6 |

| | | | | | | | | | | |
|---|---|---|---|---|---|---|---|---|---|---|
| nd = non détectable | | | | | | | | | | |

Lors de la première étape de fermentation stabilisante, la numération de la FMAR (a) passe d'une valeur initiale (série 0) dans le lait cru de 6,5 10³ micro-organismes/mL_{;} à une valeur finale dans les milieux de fermentation à pH 5,2 (séries 1, 2, 3, 4, 5 et 6) successifs de l'ordre de 1,0 10⁹ micro-organismes/mL. La numération de la flore utile (g) passe d'une valeur initiale (série 0) dans le lait cru de 3,06 10³ micro-organismes/mL, à une valeur finale moyenne dans les milieux de fermentation à pH 5,2 (séries 1, 2, 3, 4, 5 et 6) successifs qui est stable et de l'ordre de 8,0 10⁸ micro-organismes/mL.

En outre, la numération de la flore coliforme (b) augmente significativement d'une valeur initiale (série 0) dans le lait cru de 6 micro-organismes/mL, à une valeur dans le milieu de fermentation à pH 5,2 (série 1) de la première étape de fermentation stabilisante de 2,46 10⁴ micro-organismes/mL, puis décroît dans les milieux de fermentation des étapes ultérieures de fermentation stabilisante successives (séries 2, 3, et 4) jusqu'à disparaître dans les milieux de fermentation des étapes 5 et 6. Ainsi, la proportion de bactéries coliformes (b) par rapport à la FMAR passe d'une valeur de l'ordre de 0,1% dans le lait cru, à une valeur nulle dans le levain obtenu après la cinquième étape de fermentation stabilisante.

On observe également que les bactéries *Escherichia coli* (c) ainsi que les staphylocoques (d) qui ne sont pas détectables dans ce lait cru ultra-propre, ne sont pas non plus détectables dans le levain obtenu à partir de ce lait cru ultra-propre.

On observe en outre sur la figure 6 et dans le tableau 3 que les levures (e) et les moisissures (f) disparaissent totalement des milieux de fermentation successifs lors de la quatrième étape (série 4) de fermentation stabilisante d'un traitement de stabilisation selon l'invention.

## Revendications

1. Procédé de préparation d'un levain à partir de lait cru dans lequel :
- on choisit un lait cru qui n'a subi aucun traitement physique ou thermique d'élimination ou de destruction de germes microbiologiques ;
- ledit lait cru étant choisi dans le groupe formé des laits crus dont le pH atteint, lors d'une étape, dite étape (13) de sélection, de fermentation stabilisante par fermentation lactique acidifiante du lait cru, à au moins une température, dite température de sélection, comprise entre 20°C et 42°C:
o une valeur de 5,5 en plus de 15 h, et
o une valeur minimum de 5,0 en moins de 48 h, puis,
- on réalise un traitement (15) de stabilisation de lait cru ;
o ledit traitement (15) comprenant au moins deux étapes, dites étapes de fermentation stabilisante, successives de fermentation lactique acidifiante d'un milieu de fermentation, dont une première étape de fermentation stabilisante réalisée à partir d'un milieu de fermentation comprenant ledit lait cru, et ;
o dans lequel on interrompt chaque étape de fermentation stabilisante dudit traitement lorsque le pH du milieu de fermentation atteint une valeur comprise entre 4,6 et 6,0 ;
o dans lequel on réalise au moins une étape, dite étape ultérieure de fermentation stabilisante, après la première étape de fermentation stabilisante, à partir d'un milieu de fermentation formé d'une quantité d'un milieu de culture liquide et d'une quantité d'un milieu de fermentation, dit milieu de fermentation précédent, obtenu à l'issue d'une étape précédente de fermentation stabilisante dans un rapport volumique de la quantité de milieu de fermentation précédent sur la quantité de milieu de fermentation compris entre 0,5% et 25% ;
o chaque étape de fermentation stabilisante étant réalisée à une température prédéterminée comprise entre 18°C et 35°C, et ;
o chaque étape de fermentation stabilisante étant interrompue par refroidissement du milieu de fermentation à une température inférieure à +4 °C.

2. Procédé selon la revendication 1, **caractérisé en ce qu'**on réalise ladite première étape de fermentation stabilisante sans ajout ni retranchement artificiel d'espèces microbiologiques au/du lait cru.

3. Procédé selon l'une des revendications 1 ou 2, **caractérisé en ce qu'**on interrompt la première étape de fermentation stabilisante après une durée de fermentation lactique acidifiante du milieu de fermentation comprise entre 12 h et 36 h.

4. Procédé selon l'une des revendications 1 à 3, **caractérisé en ce que** le milieu de fermentation (16) de la première étape (17) de fermentation stabilisante est un lait cru issu de mammifère -notamment un lait cru choisi dans le groupe formé du lait cru issu de vache, du lait cru issu de brebis, du lait cru issu de chèvre et du lait cru issu de bufflonne-.

5. Procédé selon l'une des revendications 1 à 4, **caractérisé en ce que** le rapport volumique de la quantité de milieu de fermentation précédent sur la quantité de milieu de fermentation est compris entre 1 % et 5 %, en particulier de l'ordre de 2 %.

6. Procédé selon l'une des revendications 1 à 5, **caractérisé en ce que** le milieu de culture liquide est un jus lactosé stérilisé et osmosé contenant entre 4% et 20% en masse d'extrait sec, notamment 12% en masse d'extrait sec.

7. Procédé selon l'une des revendications 1 à 6, **caractérisé en ce que** le pH du milieu de culture liquide est compris entre 6,5 et 7,0.

8. Procédé selon l'une des revendications 1 à 7, **caractérisé en ce que** ledit milieu de fermentation précédent présente une valeur de pH comprise entre 4,6 et 6,0 et contient entre 5 10⁷ et 5 10⁹ micro-organismes/mL.

9. Procédé selon l'une des revendications 1 à 8, **caractérisé en ce qu'**on choisit le lait cru dans le groupe formé des laits crus dont le pH atteint, lors de l'étape (13) de sélection réalisée aux températures de sélection de 27°C et de 40°C :
o une valeur de 5,5 en plus de 15 h, et
o une valeur minimum de 5,0 en moins de 48 h.

## Patentansprüche

1. Verfahren zur Herstellung eines Fermentationsmittels aus Rohmilch, wobei:
- eine Rohmilch gewählt wird, die keiner physischen oder thermischen Behandlung zur Entfernung oder zur Zerstörung von mikrobiologischen Keimen unterzogen wurde;
- die Rohmilch ausgewählt wird aus der Gruppe, gebildet aus Rohmilchsorten, deren pH bei einem Schritt, genannt Schritt (13) des Auswählens, des stabilisierenden Fermentierens durch ansäuernde Milchsäurefermentierung der Rohmilch bei mindestens einer Temperatur, genannt Auswahltemperatur, die zwischen 20 °C und 42 °C liegt, Folgendes erreicht:
- einen Wert von 5,5 in mehr als 15 Stunden, und
- einen minimalen Wert von 5,0 in weniger als 48 Stunden, dann
- eine Behandlung (15) zur Stabilisierung der Rohmilch durchgeführt wird,
- wobei die Behandlung (15) mindestens zwei aufeinanderfolgende Schritte, genannt Schritte des stabilisierenden Fermentierens, der ansäuernden Milchsäurefermentierung eines Fermentierungsmediums umfasst, wobei ein erster Schritt des stabilisierenden Fermentierens, der ausgehend von einem Fermentierungsmedium durchgeführt wird, die Rohmilch enthält, und
- wobei jeder Schritt des stabilisierenden Fermentierens der Behandlung unterbrochen wird, wenn der pH des Fermentierungsmediums einen Wert erreicht, der zwischen 4,6 und 6,0 liegt;
- wobei mindestens ein Schritt, genannt letzter Schritt des stabilisierenden Fermentierens, nach dem ersten Schritt des stabilisierenden Fermentierens durchgeführt wird, ausgehend von einem Fermentierungsmedium, das aus eine Menge eines flüssigen Kulturmediums und einer Menge eines Fermentierungsmediums gebildet wird, genannt vorhergehendes Fermentierungsmedium, erhalten am Ausgang eines vorhergehenden Schritts des stabilisierenden Fermentierens in einem Volumenverhältnis der Menge des vorhergehenden Fermentierungsmediums zur Menge des Fermentierungsmediums, das zwischen 0,5 % und 25 % liegt;
- wobei jeder Schritt des stabilisierenden Fermentierens bei einer vorbestimmten Temperatur durchgeführt wird, die zwischen 18 °C und 35 °C liegt; und
- jeder Schritt des stabilisierenden Fermentierens durch Abkühlung des Fermentierungsmediums auf eine Temperatur von weniger als +4 °C unterbrochen wird.

2. Verfahren nach Anspruch 1, **dadurch gekennzeichnet, dass** der erste Schritt des stabilisierenden Fermentierens ohne künstliche Zugabe oder Entfernung von mikrobiologischen Arten zur/von der Rohmilch durchgeführt wird.

3. Verfahren nach einem der Ansprüche 1 oder 2, **dadurch gekennzeichnet, dass** der erste Schritt des stabilisierenden Fermentierens nach einer Dauer der ansäuernden Milchsäurefermentierung des Fermentierungsmediums unterbrochen wird, die zwischen 12 Stunden und 36 Stunden liegt.

4. Verfahren nach einem der Ansprüche 1 bis 3, **dadurch gekennzeichnet, dass** das Fermentierungsmedium (16) des ersten Schritts (17) des stabilisierenden Fermentierens eine Rohmilch ist, die von einem Säugetier stammt, insbesondere eine Rohmilch, ausgewählt aus der Gruppe, gebildet aus Rohmilch, die von der Kuh stammt, Rohmilch, die vom Schaf stammt, Rohmilch, die von der Ziege stammt, und Rohmilch, die vom Büffel stammt.

5. Verfahren nach einem der Ansprüche 1 bis 4, **dadurch gekennzeichnet, dass** das Volumenverhältnis der Menge des vorhergehenden Fermentierungsmediums zur Menge des Fermentierungsmediums zwischen 1 % und 5 % liegt, insbesondere im Bereich von 2 %.

6. Verfahren nach einem der Ansprüche 1 bis 5, **dadurch gekennzeichnet, dass** das flüssige Kulturmedium ein sterilisierter und osmotierter Laktosesaft ist, umfassend zwischen 4 und 20 Masseprozent Trockenextrakt, insbesondere 12 Masseprozent Trockenextrakt.

7. Verfahren nach einem der Ansprüche 1 bis 6, **dadurch gekennzeichnet, dass** der pH des flüssigen Kulturmediums zwischen 6,5 und 7,0 liegt.

8. Verfahren nach einem der Ansprüche 1 bis 7, **dadurch gekennzeichnet, dass** das vorhergehende Fermentierungsmedium einen pH-Wert aufweist, der zwischen 4,6 und 6,0 liegt, und zwischen 5 10⁷ und 5 10⁹ Mikroorganismen/mL enthält.

9. Verfahren nach einem der Ansprüche 1 bis 8, **dadurch gekennzeichnet, dass** die Rohmilch ausgewählt wird aus der Gruppe, gebildet aus Rohmilchsorten, deren pH während des Schritts (13) des Auswählens, durchgeführt bei den Auswahltemperaturen von 27 °C und 40 °C, Folgendes erreicht:
- einen Wert von 5,5 in mehr als 15 Stunden, und
- einen minimalen Wert von 5,0 in weniger als 48 Stunden.

## Claims

1. Method for preparing a leaven using raw milk, in which:
- a raw milk is chosen which has undergone no physical or thermal treatment of elimination or of destruction of microbiological germs ;
- said raw milk is chosen from the group consisting of raw milks whose pH, in a step, named the selection step (13), of stabilising fermentation by acidifying lactic fermentation of the raw milk at at least one temperature, named the selection temperature, comprised between 20°C to 42°C is capable of reaching:
∘ a value of 5.5 in more than 15 hours, and
∘ a minimum value of 5.0 in less than 48 hours, and then,
- a raw milk stabilisation treatment (15) is carried out;
∘ said treatment (15) comprising at least two steps, named the stabilising fermentation steps, succeeding the acidifying lactic fermentation of a fermentation medium, which first stabilising fermentation step is carried out using a fermentation medium comprising said raw milk, and;
∘ in which each stabilising fermentation step of said treatment is interrupted when the pH of the fermentation medium reaches a value comprised between 4.6 to 6.0;
∘ in which at least one step, named a subsequent stabilising fermentation step, is carried out after the first stabilising fermentation step, using a fermentation medium formed of a quantity of a liquid culture medium and a quantity of a fermentation medium, named the preceding fermentation medium, obtained at the end of a preceding stabilising fermentation step, in a ratio by volume of the quantity of preceding fermentation medium to the quantity of fermentation medium comprised between 0.5% to 25%,
∘ each stabilising fermentation step is carried out at a predetermined temperature comprised between 18°C and 35°C, and
∘ each stabilising fermentation step is interrupted by cooling the fermentation medium to a temperature below +4°C.

2. Method according to Claim 1, **characterized in that** said first stabilising fermentation step is carried out without artificially adding or removing microbiological species to/from the raw milk.

3. Method according to one of Claims 1 to 2, **characterized in that** the first stabilising fermentation step is interrupted after an acidifying lactic fermentation time of the fermentation medium comprised between 12 hours to 36 hours.

4. Method according to one of Claims 1 to 3, **characterized in that** the fermentation medium (16) of the first stabilising fermentation step (17) is a raw milk derived from a mammal, especially a raw milk chosen from the group consisting of raw milk derived from a cow, raw milk derived from a ewe, raw milk derived from a goat and raw milk derived from a buffalo.

5. Method according to one of Claims 1 to 4, **characterized in that** in a ratio by volume of the quantity of preceding fermentation medium to the quantity of fermentation medium is comprised between 1% and 5 %, in particular in the region of 2%.

6. Method according to one of Claims 1 to 5, **characterized in that** the liquid culture medium is a sterilised and osmosed lactose-containing liquor having a solids content comprised between 4 to 20 wt.%, especially 12 wt.%.

7. Method according to one of Claims 1 to 6, **characterized in that** the pH of the liquid culture medium is comprised between 6.5 and 7.0.

8. Method according to one of Claims 1 to 7, **characterized in that** said preceding fermentation medium has a pH value comprised between 4.6 to 6.0 and contains from 5 10⁷ to 5 10⁹ microorganisms/ml.

9. Method according to one of Claims 1 to 8, **characterized in that** the raw milk is chosen from the group consisting of raw milks whose pH, in a step (13) at the selection temperatures of 27°C and 40°C, is capable of reaching
∘ a value of 5.5 in more than 15 hours and
∘ a minimum value of 5.0 in less than 48 hours.
